(19) Europäisches Patentamt
European Patent Office
Office européen des brevets

(11) EP 1 407 784 B1

(12) **EUROPEAN PATENT SPECIFICATION**

(45) Date of publication and mention of the grant of the patent:
**24.11.2010 Bulletin 2010/47**

(21) Application number: **02738789.3**

(22) Date of filing: **24.06.2002**

(51) Int Cl.:
***A61K 31/05*** (2006.01) ***A61K 31/167*** (2006.01)
***A61K 31/573*** (2006.01) ***A61K 45/06*** (2006.01)
***A61P 35/00*** (2006.01)

(86) International application number:
**PCT/JP2002/006260**

(87) International publication number:
**WO 2003/000290 (03.01.2003 Gazette 2003/01)**

(54) **ANTITUMOR AGENTS**

ANTITUMORALE MITTEL

AGENTS ANTITUMORAUX

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.06.2001 JP 2001191067**

(43) Date of publication of application:
**14.04.2004 Bulletin 2004/16**

(73) Proprietor: **Ajinomoto Co., Inc.**
**Tokyo 104-8315 (JP)**

(72) Inventors:
- **NIHEI, Yukio**
**Kawasaki-shi,**
**Kanagawa 210-0801 (JP)**
- **MORINAGA, Yoshihiro**
**Kawasaki-shi,**
**Kanagawa 210-0801 (JP)**
- **SUZUKI, Manabu**
**Kawasaki-shi,**
**Kanagawa 210-0801 (JP)**
- **SUGA, Yasuyo**
**Kawasaki-shi,**
**Kanagawa 210-0801 (JP)**

(74) Representative: **Nicholls, Kathryn Margaret et al**
**Mewburn Ellis LLP**
**33 Gutter Lane**
**London**
**EC2V 8AS (GB)**

(56) References cited:
**WO-A-98/18490** **WO-A1-98/32440**
**WO-A1-99/51246** **WO-A2-97/05870**

- **WEISS R.B. ET AL.: 'Hypersensitivity reactions from taxol' JOURNAL OF CLINICAL ONCOLOGY vol. 8, no. 7, 1990, pages 1263 - 1268, XP000654549**
- **DATABASE MEDLINE [Online] ECKHARDT S. ET AL.: 'The effect of docetaxel on malignant tumors', XP002957137 Database accession no. 1998240262 & IRV. HETIL. vol. 139, no. 15, 1998, pages 867 - 872**
- **BROWMAN GEORGE P. ET AL.: 'Modified adriamycin-vincristine-dexamethasone(m-VAD) in primary refractory and relapsed plasma cell myeloma: an NCI(Canada) pilot study' BRITISH JOURNAL OF HAEMATOLOGY vol. 82, 1992, pages 555 - 559, XP002957138**

EP 1 407 784 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

Field of the Invention

The present invention relates to a novel anti-tumor agent, and more particularly it relates to an anti-tumor agent as a combination of a tubulin-polymerization inhibiting active substance having anti-tumor activity with an anti-inflammatory active substance according to the claims. Since the use of an anti-tumor medical agent (anti-tumor agent) containing a tubulin polymerization-inhibitory active substance in combination with an anti-inflammatory active substance can significantly increase the lethal administration dosage of a tubulin polymerization-inhibitory active substance and at the same time can maintain its pharmaceutically effective dosage at a level substantially comparable to that for non-combined administration, the safety zone for the medical agent can be expanded. Also, the toxicity of the tubulin polymerization-inhibitory substance at the pharmaceutically effective dosage can be significantly improved at the same time. As a result, the usefulness as an anti-tumor agent to be administered by medical doctors, et al can be remarkably expanded, and the burden to patients et al can be reduced.

According to the present invention, an anti-tumor pharmaceutical (pharmaceutical preparation) containing a tubulin polymerization-inhibitory active substance having anti-tumor activity selected from the group consisting of combretastatins and stilbenes to be used in combination with an anti-inflammatory active substance selected from the group consisting of dexamethasone, esters and salts thereof, and a toxicity reducing agent to be used for an anti-tumor pharmaceutical containing the tubulin polymerization-inhibitory active substance selected from the group consisting of combretastatins and stilbenes, wherein the agent comprises an anti-inflammatory active substance selected from the group consisting of dexamethasone, esters and salts thereof, are also provided.

The present invention has utility in the field of anti-tumor treatment, e.g. in methods for a medical treatment and an improvement of tumors, a prevention of progression of tumor, and a prevention of tumor. It relates to uses of the above-mentioned 2 effective components for a medical product such as an anti-tumor agent; and to a combination of the above-mentioned 2 effective components wherein the two components are to be used as a medical product such as an anti-tumor agent, simultaneously or separately.

Background Art

One of anti-tumor agents expected for their development relates to the development of medical agents (anti-tumor agents), now under way, which contain a tubulin polymerization-inhibitory active substance as an effective component. (Refer to Biochem. Mol. Biol. Int. 25 (6), 1153-1159 (1995); Br. J. Cancer 71 (4), 705-711 (1995); J. Med. Chem. 34 (8), 2579-2588 (1991); Biochemistry 28 (17), 6904-6991 (1989); US Patent No. 5,561,122; Japanese Laid-Open Patent Application JP-A-07-228,558(1995); Japanese Laid-Open Patent Application JP-A-08-301,831(1996); etc.).

As a result of detailed studies about the medical agent containing a tubulin polymerization-inhibitory active substance, the present inventors have predicted, among others, the possibility of expanding the utility of this medical agent by maintaining the pharmaceutically effective dosage, increasing the lethal dosage and improving the toxicity at the pharmaceutically effective dosage, because the administration of this medical agent has been restricted in respect to the safety zone (a ratio between the lethal administration dosage and the pharmaceutically effective dosage) and the toxicity at the pharmaceutically effective dosage, and thus have been engaged in research and examination for the creation of a medical agent by maintaining the pharmaceutically effective dosage of the effective component in the anti-tumor agent and simultaneously increasing the lethal dosage so that the safety zone for its administration can be expanded, and the toxicity level at the pharmaceutical effective dosage can be reduced, in order to expand its utility as a medical agent, and also in order to reduce burden to patients et al.

Under such situations, the development of an anti-tumor agent which retains the effectiveness (medical effect) of an anti-tumor agent containing a tubulin polymerization-inhibitory active substance as an effective component but which is improved only on the toxicity has been desired.

Problem to be Solved by the Invention

The problem to be solved according to the present invention is, when a tubulin polymerization-inhibitory active substance selected from the group consisting of combretastatins and stilbenes is used as an effective component in an anti-tumor agent, to develop a medical agent (anti-tumor agent) which can maintain its pharmaceutically effective dosage, but at the same time can significantly increase on the lethal dosage and improve toxicity at the pharmaceutically effective dosage of the tubulin polymerization inhibitory active substance.

Solution for Problem

In order to solve the problem, the present inventors have mainly examined combretastatins and stilbenes as a tubulin polymerization-inhibitory active substance which can be utilized as an effective component in such an anti-tumor agent. The inventors have made intensive research works to find out a possible method for retaining the pharmaceutically effective dosage while increasing the lethal dosage, and also for improving various kinds of toxicity caused by administering the pharmaceutically effective dosage, particularly gastroinestinal toxicity, hepatic toxicity and cardiovascular toxicity. This work has resulted in a finding that the combined use of an anti-inflammatory active substance, particularly an anti-inflammatory active steroid substance selected from the group consisting of dexamethasone, esters and salts thereof, can significantly increase the lethal administration dosage of the tubulin polymerization-inhibitory active substance selected from the group consisting of combretastatins and stilbenes, favorably to approximately double the original lethal dosage, and can significantly lower toxicity, particularly, its gastrointestinal toxicity, hepatic toxicity and cardiovascular toxicity, while the pharmaceutically effective dosage can be retained substantially at the same level to that without the combined use. Thus, its safety zone as a medical agent can be remarkably expanded and the toxicity can be significantly improved within the pharmaceutically effective dosage range, the present invention eventually having been completed based on various knowledge including this finding.

Namely, the present invention relates to an anti-tumor agent comprising at least a tubulin polymerization-inhibitory active substance having anti-tumor activity selected from the group consisting of combretastatins and stilbenes and at least an anti-inflammatory active substance selected from the group consisting of dexamethasone, esters and salts thereof (which covers the combination of both the substances) [an anti-tumor agent according to the present invention].

The anti-tumor agent according to the present invention may be in the form of a pharmaceutical (pharmaceutical preparation) containing at least a tubulin polymerization-inhibitory active substance selected from the group consisting of combretastatins and stilbenes and an anti-inflammatory active substance selected from the group consisting of dexamethasone, esters and salts thereof in a single pharmaceutical (pharmaceutical preparation), or may be in the form of a set of 2 pharmaceuticals (pharmaceutical preparations), for example; an anti-inflammatory agent and an anti-tumor pharmaceutical (pharmaceutical preparation) containing the tubulin polymerization-inhibitory active substance, or in the form of different pharmaceuticals (pharmaceutical preparations) wherein 2 kinds of the pharmaceuticals (pharmaceutical preparations) are used in combination.

The tubulin polymerization-inhibitory active substance is selected from the group consisting of combretastatins and stilbenes.

Various reports have been made on combretastins and stilbenes having anti-tumor activity (refer to J. Med. Chem. 41: 3022-3032 (1998); Bioorg. Med. Chem. Lett. 8:3153-3158 (1998); Bioorg. Med. Chem. Lett. 8:3371-3374 (1998); US Patent No. 5,561,122; US Patent No. 5,430,062; Japanese Laid-Open Patent Application JP-A-07-228,558 (1995); Japanese Laid-Open Patent Application JP-A-08-301,831(1996), the WO93/23,357 specification, the WO99/51,246 specification; etc.), and those substances described therein can be utilized according to the present invention. As a typical example of the derivatives, (Z)-N-[2-methoxy-5-[2-(3,4,5-trimethoxyphenyl)vinyl]phenyl]-L-serinamide hydrochloride (hereinafter called "AC-7700) may be cited.

Some of the substances cited in WO 00/48,606 specification as having tubulin polymerization-inhibitory activity can be used as a tubulin polymerization-inhibitory active substance according to the present invention.

The anti-inflammatory active substance to be used according to the present invention is selected from the group consisting of Dexamethasone and derivatives thereof, namely esters and salts thereof, particularly Dexamethasone, phosphate ester and salts (such as sodium salt). Conveniently, a commercial distributed anti-inflammatory agent can be procured for the use on the market.

A pharmaceutical (pharmaceutical preparation) containing the tubulin polymerization-inhibitory active substance as defined above (an anti-tumor pharmaceutical (pharmaceutical preparation)) and a pharmaceutical (pharmaceutical preparation) containing an anti-inflammatory active substance as defined above (an anti-inflammatory agent) can be simultaneously administered or can be administered at different times. Accordingly, these pharmaceuticals (pharmaceutical preparations) may be in the form for respective administrations or in the form of a single pharmaceutical (pharmaceutical preparation). Both the pharmaceuticals may be in different forms for respective administration, and in this case, both the pharmaceuticals have to constitute respectively different pharmaceuticals.

The tubulin polymerization-inhibitory active substance can be used in the form of an anti-tumor pharmaceutical (pharmaceutical preparation; pharmaceutical agent) and the anti-inflammatory active substance can be used in the form of an anti-inflammatory agent.

According to another mode, the present invention relates to an anti-tumor pharmaceutical (pharmaceutical preparation; pharmaceutical agent) containing a tubulin polymerization-inhibitory active substance having anti-tumor activity as defined above, wherein the anti-tumor pharmaceutical is to be used in combination with or as a set with an anti-inflammatory active substance as defined above (an anti-tumor pharmaceutical (pharmaceutical preparation; pharmaceutical agent) according to the present invention).

According to further another mode, the present invention relates to a toxicity-reducing agent to be used for an anti-tumor pharmaceutical (pharmaceutical preparation; pharmaceutical agent) containing a tubulin polymerization-inhibitory active substance as defined above, wherein the toxicity-reducing agent comprises an anti-inflammatory active substance as defined above (a toxicity-reducing agent according to the present invention).

The present invention covers first of all the anti-tumor agent according to the present invention, but it additionally covers the 2 different modes of inventions. Those agents according to the 2 different modes of invention are substantially same with the anti-tumor agent according to the present invention in a sense that both the 2 different modes of invention are related to a medical agent wherein 2 kinds of effective components: a tubulin polymerization-inhibitory active substance having anti-tumor activity as defined above and an anti-inflammatory active substance as defined above are combined, and therefore, can be easily worked based on explanations about the anti-tumor agent according to the present invention. The term of "an anti-tumor pharmaceutical (pharmaceutical preparation; pharmaceutical agent) " is a term used for its differentiation from the "anti-tumor agent" according to the present invention, and any and all medical agents containing the tubulin polymerization-inhibitory active substance as defined above and used for the therapy, betterment or other treatments of tumors are equally and completely covered under the term, regardless of whatever names those medical agents are termed, including anti-tumor agents.

According to the present invention, an anti-tumor medical agent can be, for example, a combination of the 2 kinds of the effective components in respectively different pharmaceutical forms, and moreover in the forms which can be administered at the same time or different times. Also, these 2 effective components may be included in the same pharmaceutical (pharmaceutical preparation; pharmaceutical agent) which allows individual administration by pharmaceutical units. Even in such modes of applications, medical agents containing at least the 2 kinds of the effective components are also covered by the anti-tumor agent according to the present invention.

Consequently, it is not necessary that plural pharmaceuticals respectively containing at least the 2 kinds of the effective components to be used according to the present invention are jointly and fixedly contained in a specific package or container, and even when these pharmaceuticals are respectively and independently present, these pharmaceuticals shall be covered by the anti-tumor agent according to the present invention as far as these pharmaceuticals are used for the same purpose as according to the present invention (the realization of an anti-tumor effect).

Furthermore, although it is indispensable that the anti-tumor agent according to the present invention contains the 2 kinds of the effective components which are used in an appropriate combination, further different effective components [components having the same medical effect (anti-tumor components) or components having a different medical effect, components for enhancing an intended medical effect, components for the further reduction of toxicity (a side effect), and other components] may be used for combination or inclusion in pharmaceuticals according to the present invention, as far as these additional components do not hinder the effect of the present invention. In the preparation of pharmaceuticals, additive components required can be appropriately selected and used for the pharmaceutical preparation.

According to further another mode, the present invention relates to uses of a tubulin polymerization-inhibitory active substance having anti-tumor activity as defined above and an anti-inflammatory active substance as defined above for a medical product such as an anti-tumor agent. The tubulin polymerization-inhibitory active substance having anti-tumor activity and the anti-inflammatory active substance can be used individually in the different pharmaceutical preparation forms. The form for uses in the medical product as such can be selected from various forms in the above-mentioned anti-tumor agent according to the present invention, the anti-tumor pharmaceutical preparation (agent), and the toxicity-reducing agent.

According to further another mode, the present invention relates to a combination of a tubulin polymerization-inhibitory active substance having anti-tumor activity as defined above with an anti-inflammatory active substance as defined above wherein the two substances are used as a medical product such as an anti-tumor agent, simultaneously or separately.

Brief Explanation of Drawings

[Figure 1]
Figure 1 shows results from the toxicity test with tumor-bearing rats in Example 1 (Scheffe's F test; *p<0.05, **p<0.01)
F344 rats subcutaneously transplanted MT-9 tumor / Dexamethasone (1mg/kg)/AC-7700 (10mg/kg); Blood biochemical indices: GPT; ■:-DEX; □:+DEX.
[Figure 2]
Figure 2 shows results from the toxicity test with tumor-bearing rats in Example 1 (Scheffe's F test; *p<0.05).
F344 rats subcutaneously transplanted MT-9 tumor / Dexamethasone (1mg/kg)/AC-7700 (10mg/kg); Blood biochemical indices: CPK; ■ :-DEX; □ :+DEX.

Mode of Working the Invention

In the following, the present invention shall be explained mainly on the mode as an anti-tumor agent according to the present invention, but other modes of the present invention can be similarly understood based on this explanation because the same 2 kinds of medical components: a tubulin polymerization-inhibitory active substance having anti-tumor activity and an anti-inflammatory active substance are combined in all these modes for working the present invention.

The anti-tumor agent according to the present invention is a medical agent which contains at least said 2 kinds of effective components, plural components being capable to be administered at the same or different times, the 2 kinds of components being administered in the form of a same pharmaceutical or different pharmaceuticals for combined use for the broad purpose of tumor suppression, such as the betterment, prevention, therapy, growth inhibition, of tumors in mammals, particularly human. As described above, as far as this effect can be achieved, it can additionally contain or be combined with other medical components, and it can also contain other components which are required for pharmaceutical preparation.

As explained above, the tubulin polymerization-inhibitory active substance to be used according to the present invention are selected from known tubulin polymerization-inhibitory active substances having anti-tumor activity, being combretastatins or stilbenes, and the below described stilbenes can be cited as particularly preferable ones.

Examples of the stilbenes to be used according to the present invention may include compounds having the fundamental skeleton of cis-stilbene, which exhibit the in-vitro activities of tubulin polymerization inhibition and anti-tumor. As for anti-tumor activity, those compounds particularly exhibiting the activity of tumor cell growth inhibition are preferable. Derivatives which are converted to a stilbene in an animal body may also be used. Provided a stilbene can exhibit the intended anti-tumor activity according to the present invention when used in an animal body, it may be used. It may be a salt, ester, solvate such as hydrate, as far as it is a pharmaceutically acceptable derivative.

Typical stilbenes having the fundamental skeleton of cis-stilbene include compounds preferably represented by the following formulae (1) and (2). These compounds include those in the form of various salts, hydrates and solvates, and particularly those in pharmaceutically acceptable forms.

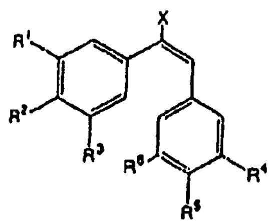

(1)

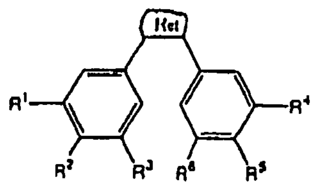

(2)

wherein $R^1$, $R^2$ and $R^3$ respectively and independently denote a lower alkoxy group, $R^4$, $R^5$ and $R^6$ respectively and independently denote a hydrogen atom, a halogen atom (fluorine, chlorine, etc.), any of substitution groups including nitro, hydroxy, lower alkoxy, phosphate ester (a substitution group formed by phosphoric ester formation with a hydroxy group: $-OP_3H_2$, hereunder meaning the same), phosphate amide (a substitution group formed by phosphoric amide formation with an amino group: $-NHPO_3H_2$, hereunder meaning the same), amino lower alkoxy, lower alkyl amino lower alkoxy, di-lower alkyl amino lower alkoxy, mercapto, lower alkyl thio, amino, lower alkyl amino, di-lower alkyl amino, lower alkyl, amino lower alkyl, trifluoromethyl, lower alkanoyl, lower alkanoyl amino and amino acid acyl amino, X denotes a hydrogen atom or a nitrile group, and Het denotes a heterocycle.

The lower alkyl and lower alkoxy groups are respectively to have 1 ~5 carbon atoms. Also, the lower alkanoyl group is to have 2~6 carbon atoms.

An amino acid acyl group in the amino acid acyl amino group is an acyl group derived from an amino acid, and

examples of the amino acid may include α-amino acid, β-amino acid and γ-amino acid. Preferable examples of the amino acid may include glycine, alanine, leucine, serine, lysine, glutamic acid, aspartic acid, threonine, valine, isoleucine, ornithine, glutamine, asparagine, tyrosine, phenyl alanine, cystine, methionine, alginine, β-alanine, tryptophan, proline, histidine, etc. Particularly preferable in respect of the pharmaceutical effect and safety are threonine and serine. These amino acids may be of any of L-, D- and DL-forms, but the L-form is preferable.

Examples of the heterocycle may include a tetrazole ring, a thiazole ring. When the heterocycle is a thiazole ring, it may be substituted. Examples of a substitution group in this case may include lower alkyl, amino, mono-lower alkyl amino, di-lower alkyl amino, hydrazino, halogen atom (fluorine, chlorine) and lower alkoxy. Incidentally, the lower alkyl and lower alkoxy groups are respectively to have 1~5 carbon atoms.

As described above, a stilbene to be used according to the present invention may be a compound having a cis-stilbene skeleton, and exhibiting tubulin polymerization-inhibitory activity and anti-tumor activity. As a specific example of the stilbene derivative, combretastatin-A4 may be cited, but the stilbene is not particularly restricted, and may include any stilbene capable of inhibiting tumor growth, such as those which have been disclosed in prior art publications, for example, patent gazettes (refer to US Patent Nos. 4,996,237; 5,561,122 and 5,430,062; and Japanese Laid-Open Patent Applications JP-A-07-228,558(1995); JP-A-08-301,831(1996) and JP-A-10-81,673(1998)).

The stilbenes can be manufactured according to known technology including manufacturing processes disclosed in the above described publications. Stilbenes to be developed in future may be also manufactured and utilized in the same manner as described above.

Stilbenes to be used according to the present invention may include those in the form of salts, esters and solvates such as hydrates.

More preferable stilbenes to be used according to the present invention are represented by the following formula (1'):

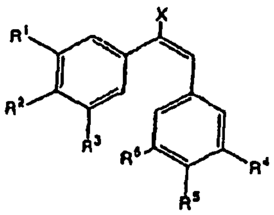

(1')

In the formula (1'), $R^1$, $R^2$, $R^3$ and $R^5$ respectively denote a methoxy group, $R^4$ denotes either an amino group or an amino acid acyl amino group, and $R^6$ and X respectively denote a hydrogen atom.

Among compounds represented by the formula (1'), a compound represented by the formula (3) [(Z)-N-[2-methoxy-5-[2-(3,4,5-trimethoxyphenyl) vinyl] phenyl]-L-serina mide ] is particularly preferable [which is hereunder called Compound (3)]. The Compound (3) may be in the form of a salt, e.g. hydrochloride (AC-7700), acetate, or methane sulfonate.

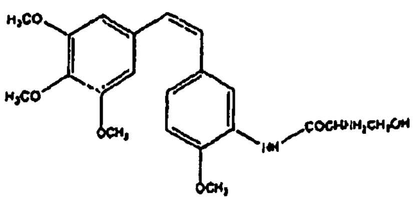

(3)

The manufacture of Compound (3) (including pharmaceutically acceptable salts, hydrates and solvates thereof) as well as the manufacture of orally or non-orally administered pharmaceutical compositions containing Compound (3) and an inert, pharmaceutically acceptable carriers or diluent have been disclosed in Japanese Laid-Open Patent Application JP-A-08-301,831(1996) in a broad range, which can be referred to for the manufacture thereof.

The compound can be used, as a medical agent according to the present invention, in the form of a pharmaceutical (pharmaceutical preparation) which contains a tubulin polymerization-inhibitory active substance having anti-tumor activity. In this case, it can be used in the form of a single pharmaceutical (pharmaceutical preparation) containing the tubulin polymerization-inhibitory active substance as a (major) effective component, or it can be also used in the form of a pharmaceutical (pharmaceutical preparation) which combines the anti-inflammatory active substance with this component (a pharmaceutical combining 2 kinds of effective components). There is no particular restriction on the form

of pharmaceutical preparations in this case. It can be used in the form of an orally administered pharmaceutical or a non-orally administered pharmaceutical, particularly in the form of an injection. Investigations for their use as an anti-tumor agent have been already under way on some of the tubulin polymerization-inhibitory active substances (for example, combretastatins) and thus, their pharmaceuticals (pharmaceutical preparations) can be easily prepared based on public knowledge obtained in relation to such investigations.

An anti-inflammatory active substance as defined above to be used in the anti-tumor agent according to the present invention can be combined and used in said pharmaceuticals, but what has been known and used as an anti-inflammatory agent can be separately used for the combined use with a pharmaceutical which contains the tubulin polymerization-inhibitory active substance having anti-tumor activity as defined above. It can be used according to a known administration method, etc. for an anti-inflammatory agent. Due to a component to be combined for use or the combined use of an anti-inflammatory active substance according to the present invention, the lethal dosage of the tubulin polymerization-inhibitory active substance is increased, preferably to about twice or more, and remarkable improvement on the toxicity at the pharmaceutically effective dosage can be achieved, particularly on gastrointestinal toxicity (betterment of diarrhea), hepatic toxicity (lowering of GPT) and cardiovascular toxicity (lowering of CPK). On the other hand, it has been found that the pharmaceutically effective dosage has not been affected by the presence or absence of the combined use according to the present invention.

As a result, when a tubulin polymerization-inhibitory active substance is used as an anti-tumor agent, a remarkable improvement can be achieved on the lethal toxicity and the toxicity at the pharmaceutically effective dosage, so that medical people such as doctors, et al can conveniently use this medical agent, and the burden on patients administered of this medical agent can be greatly reduced, too.

For example, depending upon symptoms of patients, etc., the dosage of a tubulin polymerization-inhibitory active substance, for example in the case of AC-7700 (an injection), can be preferably about 0.1~10000mg, more preferably about 0.5~1000mg, and further preferably about 1~500mg per patient a day. The dosage of an anti-inflammatory active agent to be used in combination according to the present invention, for example in the case of Dexamethasone sodium phosphate (an injection), can be preferably about 0.1~10000mg, more preferably about 0.5~1000mg and further preferably about 1~500mg per patient a day.

When these agents are to be orally administered, respective dosages can be within a range of about 2~20 times the dosage used as an injection.

The dosage for the administration of the 2 kinds of effective components according to the present invention together with other components can be optionally selected in utilization of prior art technology and measures, by referring to the above described ranges of the dosages.

The 2 kinds of components as indispensable effective components to be used according to the present invention can be respectively contained in different pharmaceutical forms and independently administered to patients who desire an anti-tumor effect, but as described above, these 2 components can contain or be combined with other pharmaceutical components to be used as a medical agent exhibiting an anti-tumor effect, and these cases are also naturally covered by the present invention as far as these medical agents exert an anti-tumor effect. A tubulin polymerization-inhibitory active substance can be combine with an anti-inflammatory active substance for medical uses, and in this case, further components can be contained or be combined with these 2 kinds of components for the use of a tubulin polymerization-inhibitory active substance so that a similar anti-tumor effect can be exerted, and such a use is also covered by the present invention.

In the preparation of pharmaceuticals, pharmacologically acceptable, various preparatory substances can be also contained (as supplemental agents). Substances for pharmaceutical preparation can be optionally selected depending upon the form of preparations, examples of which may include vehicles, diluents, additives, disintegrators, binders, coating agents, lubricants, sliding agents, smoothing agents, flavoring agents, sweeteners, solubilizers. Furthermore, specific examples of preparatory substances may include magnesium carbonate, titanium dioxide, lactose, mannitol and other saccharides, talc, milk casein, gelatin, starch, cellulose and derivatives thereof, animal and vegetable oils, polyethylene glycol, and solvents such as sterile water and mono- or polyhydric alcohols, for example, glycerol.

It is not necessary to include all the effective components to be used according to the present invention in the same single pharmaceutical, and respective components or the 2 components may be appropriately contained in one or two pharmaceuticals. In this case, the components may be prepared to various forms of pharmaceuticals, either known or to be developed in future, for example, including various administration forms such as for oral administration, abdominal administration, dermal administration, inhalation administration, intravenous administration. In order to prepare pharmaceutical components to be used according to the present invention into such various pharmaceutical forms, methods, either known or to be developed in future, may be optionally adopted.

Examples of such various forms of pharmaceuticals may include appropriate solid or liquid pharmaceutical forms, such as granules, powder, coated tablets, tablets, (micro-)capsules, depositories, syrups, juices, suspensions, emulsions, drops, injection solutions, preparations for extended release of an active substance.

Medical agents according to the present invention, prepared in pharmaceutical forms such as cited above in

the examples, should naturally contain pharmaceutically effective amounts of said components in order to achieve the intended effect.

(Anti-tumor pharmaceutical according to the present invention)

As described above, an anti-tumor pharmaceutical containing a tubulin polymerization-inhibitory active substance having anti-tumor activity as defined above, which is characterized by the combined use with an anti-inflammatory active substance as defined above, is covered by the present invention, and since this pharmaceutical is substantially same with the anti-tumor agent according to the present invention in respect to the combined use of the 2 kinds of medical components, a skilled person in the art should be able to practice this invention based on the above described detailed explanation and later described examples, as well as prior art technology.

(Toxicity-reducing agent according to the present invention)

Similarly, a toxicity-reducing agent for an anti-tumor pharmaceutical containing a tubulin polymerization-inhibitory active substance as defined above, wherein an anti-inflammatory active substrate as defined above is contained, is also covered by the present invention, and since this toxicity-reducing agent is substantially same with the anti-tumor agent according to the present invention in respect to the combined use of the 2 kinds of medical components in the same manner as described above, a skilled person in the art should be able to practice this invention based on the above described detailed explanation and later described examples, as well as prior art technology.
As described above, according to other modes, the present invention may be employed in a use for the anti-tumor treatment, which comprises administering containing a tubulin polymerization-inhibitory active substance having anti-tumor activity as defined above and an anti-inflammatory active substance as defined above to a living subject, wherein the use includes uses for a medical treatment and an improvement of tumors, a prevention of progression of tumor, and a prevention of tumor. In further modes the invention relates to uses of a tubulin polymerization-inhibitory active substance having anti-tumor activity as defined above and an anti-inflammatory active substance as defined above for a medical product such as an anti-tumor agent; and to a combination of a tubulin polymerization-inhibitory active substance having anti-tumor activity as defined above with an anti-inflammatory active substance as defined above wherein the two substances are used as a medical product such as an anti-tumor agent, simultaneously or separately.
These modes of the present invention can all be carried out readily on the basis of the descriptions about the above-mentioned anti-tumor agent, anti-tumor pharmaceutical preparation (agent), and/or toxicity-reducing agent according to the present invention or the after described Examples and the like, with reference to known art, if necessary.

Preferred Modes for Operation

In the following, the present invention shall be explained in reference to illustrative examples.

[Example 1]

(1) Tumor cell line and experimental animal

Rat tumor cell line (transplant rat strain);

Malignant fibrous histiocytoma MT-9 (F344, male)

MT-9 was obtained as in-vitro cultured cells, which were cultured in RPMI1640 medium containing 10% FBS and then, $10^7$ or more of the cells were subcutaneously transplanted to rats on their back. After tumor formation, tumor slices (about 100mg) were inoculated subcutaneously into rats using cannula for serial passage.

F344 (5 weeks old) was acquired from Charles River Japan.

(2) Medicines and administration method

As the tubulin polymerization-inhibitory active substance having anti-tumor activity, (Z)-N-{2-methoxy-5-[2-(3,4,5-trimethoxyphenyl)vinyl)phenyl]-L-serinamide hydrochloride (AC-7700) was used.
AC-7700 had been stored in a dark place at a low temperature (5°C) after the synthesis, and after weighing, it was dissolved in physiological saline immediately before the administration.

As the Dexamethasone (its derivative), "Decadron S injection" made by Banyu Pharmaceutical (Demethasone sodium phosphate, its chemical term being 16 a -methyl-9 $\alpha$-fluoroprednisolone 21-phsophate disodium salt, hereinafter simply called "Dexamethasone" or "DEX") was dilued with physiological saline immediately before the administration and was injected intravenously.

(3) Procedure for the biochemical testing of blood

Under ether anesthesia, rats were subjected to ventrotomy, and blood was sampled from inferior vena cava using heparin-containing syringes. The blood samples were centrifuged at 3000rpm for 10 minutes to recover plasma, and the GOT, GPT, CPK and LDH concentration in plasma were determined using Fuji Dry Chem. When the measurement was not conducted immediately, the plasma samples had been stored at -80°C until the determination was made.

(4) Procedure for the evaluation of in vivo pharmaceutical effect

(anti-tumor effect)

The MT-9/F344 system: Tumors serially passed by the subcutaneous transplantation were extirpated. After binding tissues and necrosis portions in the tumors had been removed, tumor tissues were minced with scissors and made pasty, and about 50 ~ 100mg of the tumor tissues were subcutaneously transplanted to F344 rats on their back (day 0).

After the tumors were multiplied to be measurable (about 1~2 weeks later), the tumor size (tumor volume) and body weight were measured and divided into tumor size-and body weight-matched groups.

The tumor sizes (tumor volumes) and body weights were measured every day from the next day to about the third day after the administration had been completed. Incidentally, the tumor volume was calculated according to the following formula:

$$\text{Tumor volume } (mm^3) = [(\text{larger diameter, mm}) \times (\text{smaller diameter, mm})]^2 \times 1/2.$$

For the determination of the anti-tumor effect, the T/C and I.R. values were calculated according to the following formula, and when the T/C value was 50% or less (the I.R. value being 50% or more) and statistically significant difference from the value for control existed, the anti-tumor effect was judged to be positive, and the anti-tumor effect on a day when the maximum anti-tumor effect was observed was determined as the pharmaceutical effect.

$$T/C(\%) = [(\text{tumor volume for the medicine administered group}) \div (\text{tumor volume for the control group})] \times 100$$

$$I.R. (\%) = 100 - T/C.$$

Weight changes were derived by the deduction of the body weight on the day when the treatment was started from the body weight on the day for evaluating the pharmaceutical effect. Incidentally, the tumor weight (g) was calculated by conversion as (tumor volume X 1/1000), and changes in the body weight excluding the tumor weight were determined based on the deduction of respective tumor weights from the body weight.

(5) Statistical analysis

The growth of rat tumors was statistically analyzed on the presumption that it does not follow normal distribution. In the comparison between the control group and treated groups administered with respective dosages (2 group comparison), the Mann-Whitney U test was used, and a P value of 0.5 or less was judged significant. In the multiple group comparison, Scheff's F test was used, and a P value of 0.05 or less was judged significant.

(6) Results

(1) Reduction of AC-7700 toxicity with Dexamethasone

MT-9 was subcutaneously transplanted to F344 rats on their back, and after the tumor was formed, the medical agent was administered. AC-7700 was administered at the pharmaceutically effective single agent dosage of 10mg/kg, and Dexamethasone was administered at the dosage of 1mg/kg on a day before the administration of AC-7700. The biochemical indices of blood were measured 6 hours after AC-7700 administration. Results are shown in Figures 1 and 2.
The results show that Dexamethasone had remarkably reduced the toxicity of AC-7700 (10mg/kg), hepatic toxicity (GPT) and cardiovascular toxicity (CPK) in tumor bearing rats.
Concerning the gastrointestinal toxicity, the combined use of Dexamethasone with AC-7700 has revealed that diarrhea induced by AC-7700 in mice was significantly improved.

(2) Influence of Dexamethasone on the pharmaceutical effect of AC-7700

The influence of Dexamethasone on the pharmaceutical effect of AC-7700 was investigated using F344 rats, subcutaneously transplanted MT-9. AC-7700 was administered at the dosage of 10mg/kg, and 1mg/kg of Dexamethasone was administered on the day before the AC-7700 administration. Both the medical agents were 3 times administered every 3 days.
AC-7700 significantly inhibited tumor growth in a single agent. The administration of AC-7700 in combination with Dexamethasone also inhibited tumor growth in the same manner (refer to Table 1). There was no significant difference between anti-tumor effect of AC-7700 and those of AC-7700 combined with Dexamathasone (Scheffe's F test).

[Table 1] Influence of Dexamethasone on the pharmaceutical effect of AC-7700

| DEX(mg/kg/day) | AC-7700(mg/kg/day) | I.R.(%) |
|---|---|---|
| 0 | 0 | 0 |
| 0 | 10 | 84** |
| 1 | 0 | 21 |
| 1 | 10 | 72** |
| (Note: Mann-Whitney's U test; **: p<0.01 vs. Control) | | |

Furthermore, in the investigation using CDF1 mice (female), Dexamethasone (5mg/kg/day administered by intravenous injection into the tail, on a day before and on the day of the AC-7700 administration), increased the maximum tolerable dose of AC-7700 (3 times subcutaneously administered every 3 days) from 43.6mg/kg/day to more than 90mg/kg/day.

Advantages of the Invention

According to the present invention, an anti-tumor agent containing a tubulin polymerization-inhibitory active substance selected from the group consisting of combretastatins and stilbens as an effective component is provided, wherein the medical agent (anti-tumor agent) in combination with an anti-inflammatory active substance selected from the group consisting of dexamethasone, esters and salts thereof can retain its pharmaceutically effective dosage but can have remarkably improved toxicity of the tubulin polymerization-inhibitory active substance and an increased the lethal dosage so that its safety zone can be expanded.
The safety zone is expanded and thus its application on by medical doctors and others in the therapy, betterment of tumors, while the burden on patients and others can be reduced.
In addition, the present invention also provides the followings:

i) A use in a method for the anti-tumor treatment, wherein the use includes uses for a medical treatment and an improvement of tumors, a prevention of progression of tumor, and a prevention of tumor;
ii) Uses of the above-mentioned 2 effective components for a medical product such as an anti-tumor agent; and
iii) A combination of the above-mentioned 2 effective components wherein the two components are used as a medical product such as an anti-tumor agent, simultaneously or separately.

Accordingly, the present invention can be carried out in the field of medical products and the like, and therefore is very useful industrially.

## Claims

1. An anti-tumor agent comprising: a tubulin polymerization-inhibitory active substance selected from the group consisting of combretastating and stilbenes; and an anti-inflammatory active substance selected from the group consisting of Dexamethasone, esters thereof and salts thereof.

2. An anti-tumor agent according to Claim 1, wherein the tubulin polymerization-inhibitory active substance is (Z)-N-[2-methoxy-5-[2-(3,4,5-trimethoxyphenyl)vinyl]phenyl]-L-serinamide or salt thereof.

3. An anti-tumor agent according to claim 1 wherein the anti-inflammatory active substance is Dexamethasone phosphate ester.

4. An anti-tumor agent according to Claim 1, wherein the tubulin polymerization-inhibitory active substance is in the form of an anti-tumor pharmaceutical preparation and the anti-inflammatory active substance is in the form of an anti-inflammatory agent.

5. An anti-tumor agent according to Claim 4, wherein the anti-tumor pharmaceutical preparation and the anti-inflammatory agent are for separate administration.

6. Use of a tubulin polymerization-inhibitory active substance selected from the group consisting of combretastating and stilbenes in the manufacture of an anti-tumor pharmaceutical preparation, which preparation is to be used in combination with an anti-inflammatory active substance selected from the group consisting of Dexamethasone and esters and salts thereof.

7. Use of an anti-inflammatory active substance selected from the group consisting of Dexamethasone and esters and salts thereof in the manufacture of a toxicity reducing agent for use in reducing the toxicity of an anti-tumour pharmaceutical agent comprising a tubulin polymerization-inhibitory active substance selected from the group consisting of combretastating and stilbenes.

8. Use of a tubulin polymerization-inhibitory active substance having anti-tumor activity selected from the group consisting of combretastatins and stilbenes and an anti-inflammatory active substance selected from the group consisting of Dexamethasone and esters and salts thereof in the manufacture of an anti-tumour agent of any one of claims 1 to 5.

9. A medical product comprising a combination of a tubulin polymerization-inhibitory active substance having anti-tumor activity selected from the group consisting of combretastatins and stilbenes with an anti-inflammatory active substance, selected from the group consisting of Dexamethasone and esters and salts thereof wherein the two substances are arranged for administration simultaneously or separately.

## Patentansprüche

1. Antitumormittel, welches enthält: eine als Inhibitor der Tubulinpolymerisation aktive Substanz, die aus der Gruppe ausgewählt ist, die aus Combretastatinen und Stilbenen besteht; und eine entzündungshemmend aktive Substanz, die aus der Gruppe ausgewählt ist, die aus Dexamethason, Estern und Salzen davon besteht.

2. Antitumormittel nach Anspruch 1, wobei die als Inhibitor der Tubulinpolymerisation aktive Substanz (Z)-N-[2-Methoxy-5-[2-(3,4,5-trimethoxyphenyl)vinyl]phenyl]-L-serinamid oder ein Salz davon ist.

3. Tubulinpolymerisation nach Anspruch 1, wobei die entzündungshemmend aktive Substanz Dexamethasonphosphatester ist.

4. Antitumormittel nach Anspruch 1, wobei die als Inhibitor der Tubulinpolymerisation aktive Substanz in der Form einer Antitumorarzneimittelzubereitung vorliegt und die entzündungshemmend aktive Substanz in der Form eines Antientzündungsmittels vorliegt.

5. Antitumormittel nach Anspruch 4, wobei die Antitumorarzneimittelzubereitung und das Antientzündungsmittel für eine getrennte Verabreichung vorgesehen sind.

6. Verwendung einer als Inhibitor der Tubulinpolymerisation aktiven Substanz, die aus der Gruppe ausgewählt ist, die aus Combretastatinen und Stilbenen besteht, bei der Herstellung einer Antitumorarzneimittelzubereitung, wobei die Zubereitung für die Verwendung in Kombination mit einer entzündungshemmend aktiven Substanz, die aus der aus Dexamethason und Estern und Salzen davon bestehenden Gruppe ausgewählt ist, vorgesehen ist.

7. Verwendung einer entzündungshemmend aktiven Substanz, die aus der aus Dexamethason und Estern und Salzen davon bestehenden Gruppe ausgewählt ist, zur Herstellung eines Mittels zum Verringern der Toxizität für die Verwendung bei der Verringerung der Toxizität eines Antitumorarzneimittels, welches eine als Inhibitor der Tubulinpolymerisation aktive Substanz, die aus der aus Combretastatinen und Stilbenen bestehenden Gruppe ausgewählt ist, umfasst.

8. Verwendung einer als Inhibitor der Tubulinpolymerisation aktiven Substanz mit Antitumoraktivität, die aus der aus Combretastatinen und Stilbenen bestehenden Gruppe ausgewählt ist, und einer entzündungshemmend aktiven Substanz, die aus der aus Dexamethason und Estern und Salzen davon bestehenden Gruppe ausgewählt ist, bei der Herstellung eines Antitumormittels nach einem der Ansprüche 1 bis 5.

9. Medizinisches Produkt, das eine Kombination einer als Inhibitor der Tubulinpolymerisation aktiven Substanz mit Antitumoraktivität, ausgewählt aus der aus Combretastatinen und Stilbenen bestehenden Gruppe, mit einer entzündungshemmend aktiven Substanz, ausgewählt aus der aus Dexamethason und Estern und Salzen davon bestehenden Gruppe, umfasst, wobei die zwei Substanzen für die gleichzeitige oder getrennte Verabreichung angeordnet sind.

**Revendications**

1. Agent antitumoral comprenant : une substance active inhibitrice de la polymérisation de la tubuline sélectionnée dans le groupe constitué des combrétastatines et des stilbènes ; et une substance active anti-inflammatoire sélectionnée dans le groupe constitué de la dexaméthasone, de ses esters et de ses sels.

2. Agent antitumoral selon la revendication 1, où la substance active inhibitrice de la polymérisation de la tubuline est le (Z)-N-[2-méthoxy-5-[2-(3,4,5-triméthoxyphényl)vinyl]phényl]-L-sérinamide ou un sel de ce dernier.

3. Agent antitumoral selon la revendication 1, où la substance active anti-inflammatoire est l'ester phosphate de dexaméthasone.

4. Agent antitumoral selon la revendication 1, où la substance active inhibitrice de la polymérisation de la tubuline se présente sous la forme d'une préparation pharmaceutique antitumorale et la substance active anti-inflammatoire se présente sous la forme d'un agent anti-inflammatoire.

5. Agent tumoral selon la revendication 4, où la préparation pharmaceutique antitumorale et l'agent anti-inflammatoire sont destinés à une administration distincte.

6. Utilisation d'une substance active inhibitrice de la polymérisation de la tubuline sélectionnée dans le groupe constitué des combrétastatines et des stilbènes dans la production d'une préparation pharmaceutique antitumorale, laquelle préparation doit être utilisée en association avec une substance active anti-inflammatoire sélectionnée dans le groupe constitué de la dexaméthasone et de ses esters et de ses sels.

7. Utilisation d'une substance active anti-inflammatoire sélectionnée dans le groupe constitué de la dexaméthasone et de ses esters et de ses sels dans la production d'un agent réduisant la toxicité pour utilisation dans la réduction de la toxicité d'un agent pharmaceutique antitumoral comprenant une substance active inhibitrice de la polymérisation de la tubuline sélectionnée dans le groupe constitué des combrétastatines et des stilbènes.

8. Utilisation d'une substance active inhibitrice de la polymérisation de la tubuline ayant une activité antitumorale sélectionnée dans le groupe constitué des combrétastatines et des stilbènes et d'une substance active anti-inflammatoire sélectionnée dans le groupe constitué de la dexaméthasone et de ses esters et de ses sels dans la production

d'un agent antitumoral selon l'une quelconque des revendications 1 à 5.

**9.** Produit médical comprenant une association d'une substance active inhibitrice de la polymérisation de la tubuline ayant une activité antitumorale sélectionnée dans le groupe constitué des combrétastatines et des stilbènes avec une substance active anti-inflammatoire, sélectionnée dans le groupe constitué de la dexaméthasone et de ses esters et de ses sels, où les deux substances sont disposées pour une administration simultanée ou distincte.

Fig.1

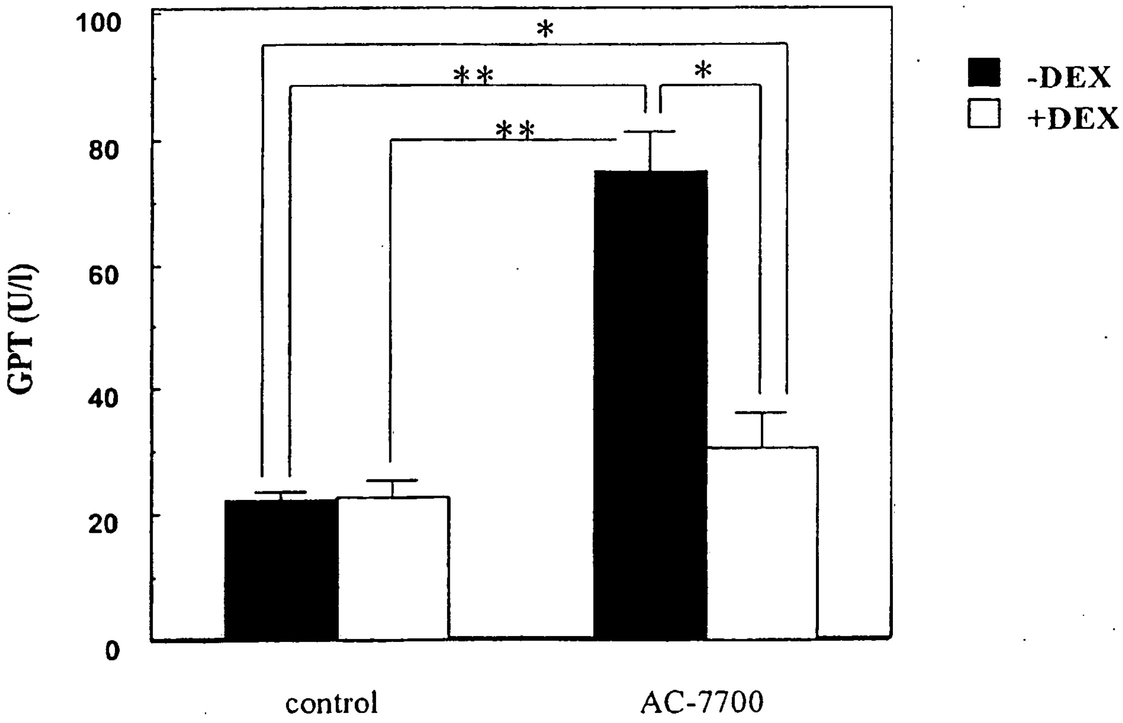

Fig.2

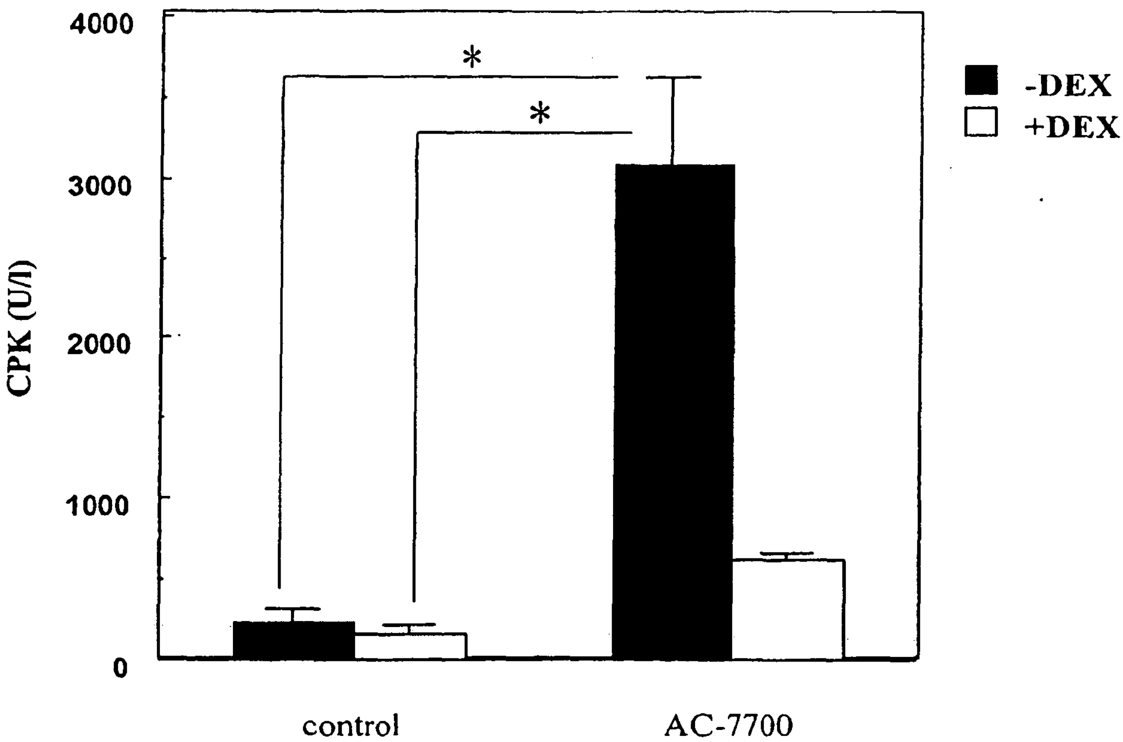

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5561122 A
- JP 7228558 A
- JP 8301831 A
- US 5430062 A
- WO 9323357 A
- WO 9951246 A
- WO 0048606 A
- US 4996237 A
- JP 10081673 A


### Non-patent literature cited in the description


- *Biochem. Mol. Biol. Int.,* 1995, vol. 25 (6), 1153-1159
- *Br. J. Cancer,* 1995, vol. 71 (4), 705-711
- *J. Med. Chem.,* 1991, vol. 34 (8), 2579-2588
- *Biochemistry,* 1989, vol. 28 (17), 6904-6991
- *J. Med. Chem.,* 1998, vol. 41, 3022-3032
- *Bioorg. Med. Chem. Lett.,* 1998, vol. 8, 3153-3158
- *Bioorg. Med. Chem. Lett.,* 1998, vol. 8, 3371-3374